# EUROPÄISCHE PATENTANMELDUNG

(11) **EP 4 446 004 A1**
(43) Veröffentlichungstag der Anmeldung: **16.10.2024**
(21) Anmeldenummer: 24168656.7
(22) Anmeldetag: 05.04.2024
(51) Int. Cl.: B01J 23/42, B01J 35/61, C10G 45/40, B01J 35/40

(54) **KATALYSATOR UND VERFAHREN ZUR HYDRIERUNG VON OLEFINEN**

(30) Priorität: 11.04.2023 EP 23167262
(71) Anmelder: Evonik Oxeno GmbH & Co. KG, 45772 Marl (DE)
(72) Erfinder: Meier, Ralf, 48324 Sendenhorst (DE); Stochniol, Guido, 45721 Haltern am See (DE); Peitz, Stephan, 45739 Oer-Erkenschwick (DE); Rix, Armin Matthias, 45770 Marl (DE)
(74) Vertreter: Evonik Patent Association

(57) **Zusammenfassung**

Gegenstand der vorliegenden Erfindung ist ein Katalysator für die Totalhydrierung eines olefinhaltigen Kohlenwasserstoffstroms. Der Katalysator umfasst ein Trägermaterial und als katalytisch aktives Metall Platin. Ein weiterer Gegenstand der vorliegenden Erfindung ist ein Verfahren zur Totalhydrierung eines olefinhaltigen Kohlenwasserstoffstroms in mindestens einem Reaktor unter Zusatz von Wasserstoff und unter Verwendung des erfindungsgemäßen Katalysators.

## Beschreibung

Die vorliegende Erfindung betrifft einen Katalysator für die Totalhydrierung eines olefinhaltigen Kohlenwasserstoffstroms. Der Katalysator umfasst ein Trägermaterial und als einziges katalytisch aktives Metall Platin. Ein weiterer Gegenstand der vorliegenden Erfindung ist ein Verfahren zur Totalhydrierung eines olefinhaltigen Kohlenwasserstoffstroms in mindestens einem Reaktor unter Zusatz von Wasserstoff und unter Verwendung des erfindungsgemäßen Katalysators.

Bei der Hydrierung werden bekanntermaßen Moleküle mit mindestens einer Doppelbindung, beispielsweise Olefine, durch die Addition von Wasserstoff zu Molekülen umgesetzt, die mindestens eine Doppelbindung weniger aufweisen. Entsprechende Verfahren sind in der petrochemischen Industrie seit vielen Jahren bekannt. Dabei werden häufig Palladium-haltige heterogene Katalysatoren eingesetzt, mit denen recht gute Hydrierergebnisse erzielt werden können. Palladium (Pd) ist hier üblicherweise das aktive Metall, welches die eigentliche Hydrierreaktion katalysiert.

In den letzten Jahren ist der Preis für Palladium stetig angestiegen. Dieser stetige Anstieg hat dazu geführt, dass auch die Palladium-haltigen Katalysatoren für den Einsatz in der Hydrierung stetig im Preis gestiegen sind. Diese Katalysatoren werden aus wirtschaftlicher Sicht also zunehmend unattraktiv. Als Alternative zu Palladium sind verschiedene hydrieraktive Metalle bekannt, beispielsweise Nickel. Problem hierbei ist, dass Nickelkatalysatoren in der Hydrierung von Olefinen recht schwer handhabbar sind.

Die Aufgabe der vorliegenden Erfindung war deshalb die Bereitstellung eines Katalysators für die Totalhydrierung eines olefinhaltigen Kohlenwasserstoffstroms, welcher die bekannten Probleme nicht aufweist. Der Katalysator soll wirtschaftlich interessant sein, aber gleichzeitig eine hohe massenspezifische Aktivität aufweisen.

Überraschenderweise wurde gefunden, dass massenspezifische Aktivität durch den Einsatz von Platin als einzigem hydrieraktiven Metall deutlich gesteigert werden kann. Dadurch wird ermöglicht, dass kostengünstigere Katalysatoren in der Hydrierung von Olefinen eingesetzt werden können.

Gegenstand der vorliegenden Erfindung ist somit ein heterogener Katalysator für die Totalhydrierung eines olefinhaltigen Kohlenwasserstoffstroms,
wobei der Katalysator ein Trägermaterial, welches aus der Gruppe bestehend aus Aluminiumoxid, Siliziumdioxid wie Kieselsäure oder Kieselgel, Kieselgur, Alumosilikate einschließlich Zeolithe, Titandioxid, Aktivkohle und Mischungen daraus, ausgewählt wird, und
ein einziges katalytisch aktives Metall in einer Menge von 0,05 bis 1 Gew.-%, bezogen auf das Gesamtgewicht des Katalysators, umfasst
wobei das katalytisch aktive Metall Platin ist.

Die vorliegende Erfindung betrifft somit einen Katalysator, der nu ein einziges katalytisch aktives Metall, hier Platin, enthält. Platin ist also das einzige hydrieraktive Metall in dem erfindungsgemäßen Katalysator. Es handelt sich also nicht um einen bimetallischen Katalysator, sondern um einen monometallischen Katalysator. Insbesondere ist der erfindungsgemäße Katalysator somit frei von Palladium oder anderen Metallen wie Zinn, Blei, Nickel. Frei von bedeutet in diesem Zusammenhang, dass diese Metalle nicht im Katalysator enthalten sind.

Liegt der Katalysator als Granulat vor, liegt die Partikelgröße (Länge der längsten Kante) des Granulats vorzugsweise im Bereich von 0,5 bis 5 mm, vorzugsweise im Bereich von 1 bis 4 mm. Liegt der Katalysator in Form von Extrudaten vor, liegt Partikelgröße (Länge der längsten Kante) der Extrudate vorzugsweise im Bereich von 0,5 bis 2,5 mm, vorzugsweise im Bereich von 1 bis 2 mm. Die Partikelgröße lässt sich mit bekannten Verfahren ermitteln. Die Partikel können auch eine sphärische Form, also eine Kugelform aufweisen. In diesem Fall beträgt der Durchmesser vorzugsweise 0,5 bis 5 mm, besonders bevorzugt 2 bis 4 mm.

Das Platin als hydrieraktives Metall liegt in den Katalysatorpartikeln des erfindungsgemäßen Katalysators vorzugsweise in einer äußeren Randzone vor, wobei die Randzonendicke kleiner 500 µm, bevorzugt kleiner 250 µm beträgt. Die Abscheidung des Metalls in die äußere Randzone kann thermisch oder durch chemische Fällung auf das Trägermaterial erfolgen. Die Bestimmung der Randzone kann mittels mikroskopischer Bildanalyse von aufgeschnittenen Katalysatorpartikeln erfolgen. Dabei kann die Dicke der Randzone mittels in das Mikroskop integrierter Software gemessen werden.

Für Katalysatoren, die kleine Mengen (z. B. < 3 Gew.-%) an Dienen oder acetylenhaltigen Komponenten zu Olefinen hydrieren sollen, werden bevorzugt Träger mit einer hohen BET-Oberfläche eingesetzt, d. h. vorzugsweise Träger mit einer BET-Oberfläche > 100 m²/g, besonders bevorzugt Träger mit einer BET-Oberfläche > 150 m²/g, um eine möglichst hohe Dispersion der Edelmetallkomponenten zu erreichen. Bei höheren Konzentrationen an Dienen oder acetylenhaltigen Komponenten werden bevorzugt Träger mit einer kleinen BET-Oberfläche eingesetzt, d. h. vorzugsweise Träger mit einer BET-Oberfläche < 100 m²/g. Geringe BET-Oberflächen weisen in der Regel eine geringe Azidität des Trägermaterials aus, so dass die Neigung zur Polymerbildung auf dem Träger reduziert wird.

Ein weiterer Gegenstand der vorliegenden Erfindung ist ein Verfahren zur Totalhydrierung eines olefinhaltigen Kohlenwasserstoffstroms, wobei das Verfahren in einem Reaktor unter Zusatz von Wasserstoff durchgeführt wird, dadurch gekennzeichnet, dass in dem Verfahren der heterogene Katalysator eingesetzt wird, der ein Trägermaterial, welches aus der Gruppe bestehend aus Aluminiumoxid, Siliziumdioxid wie Kieselsäure oder Kieselgel, Kieselgur, Alumosilikate einschließlich Zeolithe, Titandioxid, Aktivkohle oder Mischungen daraus ausgewählt wird, und Platin als katalytisch aktives Metall in einer Menge von 0,05 bis 1 Gew.-%, bevorzugt 0,1 bis 0,3 Gew.-%, jeweils bezogen auf das Gesamtgewicht des Katalysators umfasst.

Totalhydrierung im Sinne der vorliegenden Erfindung bezeichnet ein Verfahren, bei dem alle einfach oder mehrfach ungesättigten Verbindungen (Olefine, Diene, Acetylene, usw.) zu gesättigten Verbindungen, d. h. den entsprechenden Alkanen hydriert werden. Im Unterschied dazu bezeichnet die Selektivhydrierung die Hydrierung einzelner Moleküle oder einer Gruppe von Molekülen bei gleichzeitigem Erhalt anderer Moleküle oder Gruppen von Molekülen mit einer Doppelbindung. Die Selektivhydrierung nur eines Teils der anwesenden Olefine ist nicht Gegenstand der vorliegenden Erfindung. Die Totalhydrierung hat zum Ziel, dass möglichst alle Moleküle mit einer Doppelbindung im eingesetzten olefinhaltigen Kohlenwasserstoffstrom hydriert werden, die Hydrierung also quantitativ erfolgt. Aus technischen Gründen ist eine vollständige Hydrierung kaum oder nur mit hohem Aufwand zu erreichen. Der Begriff Totalhydrierung eines olefinhaltigen Kohlenwasserstoffstrom meint also erfindungsgemäß Verfahren, bei dem mindestens 99 % aller Olefine, vorzugsweise mehr als 99,5 % aller Olefine im olefinhaltigen Kohlenwasserstoffstrom hydriert werden.

Das Verfahren ist nach einer bevorzugten Ausführungsform eine Hydrierung von olefinhaltigen Kohlenwasserstoffströmen, die lineare oder verzweigte Butene umfassen (C4-Ströme), die lineare oder verzweigte Dibutene umfassen (C8-Ströme), die lineare oder verzweigte Tributene umfassen (C12-Ströme) oder die lineare oder verzweigte Tetrabutene umfassen (C16-Ströme). Für die Hydrierung dieser Moleküle werden typischerweise keine Reinströme eingesetzt, sondern technisch verfügbare Mischungen (z. B. Crack-C4-Ströme, Raffinate oder ähnliches, sowie Mischungen aus Oligomerisierungsverfahren), bei der neben den zu hydrierenden Substanzen zusätzliche Kohlenwasserstoffe vorhanden sind.

Der Wasserstoff kann bei der erfindungsgemäßem Totalhydrierung in stöchiometrischer Menge oder im leichten Überschuss zugegeben werden. Der Wasserstoff wird bei der erfindungsgemäßem Totalhydrierung vorzugsweise in einer Menge von 1,0 bis 1,5 mol Wasserstoff pro mol Olefin, besonders bevorzugt in einer Menge von 1,0 bis 1,3 mol Wasserstoff pro mol Olefin zudosiert. Bestenfalls wird der Wasserstoff in einer Menge von 1,0 bis 1,1 mol Wasserstoff pro mol Olefin zugegeben. Durch den maximal geringen Überschuss an Wasserstoff soll verhindert werden, dass durch ein zu großes Überangebot Nebenreaktionen auftreten und/oder die anderen Kohlenwasserstoffe abreagieren.

Die Totalhydrierung kann in einem breiten Temperaturbereich durchgeführt und hängt beispielsweise von der Länge der Moleküle ab. Die erfindungsgemäße Hydrierung wird vorzugsweise bei einer Temperatur zwischen 20 und 300 °C, weiterhin bevorzugt zwischen 25 und 250 °C und besonders bevorzugt zwischen 30 und 200 °C durchgeführt.

Bei der Totalhydrierung liegt vorzugsweise mindestens ein leichter Überdruck vor, um die Löslichkeit des Wasserstoffs in der flüssigen Phase zu begünstigen. Bei geringer Wasserstofflöslichkeit in der flüssigen Phase kann auch ein (sehr) hoher Druck sinnvoll sein. Die erfindungsgemäße Totalhydrierung kann in der Gasphase, in der Flüssigphase oder in einer Mischphase durchgeführt werden. Mischphase bedeutet in diesem Fall, dass der Wasserstoff nicht vollständig gelöst, sondern neben der flüssigen Phase auch eine gasförmige Phase vorliegt. In einer bevorzugten Ausführungsform der vorliegenden Erfindung sollte der Reaktordurchmesser so gewählt werden, dass Strömungsgeschwindigkeit so gering ist, dass die Flüssigkeit nicht das Gas vor sich herschiebt, sondern Gas und Flüssigkeit gemeinsam im Querschnitt vorliegen. Die Froude-Zahl der Flüssigkeit sollte dazu kleiner als 0,3 sein.

Die Hydrierung der vorliegenden Verfahren kann in jedem geeigneten Reaktor durchgeführt werden, beispielsweise in einem Rieselbettreaktor, in einem Kreislaufreaktor mit einem nachgeschaltetem Finisherreaktor oder in einem Pulse-Flow-Reaktor. Entsprechende Reaktoren sind dem Fachmann bekannt. In einer bevorzugten Ausführungsform der vorliegenden Erfindung wird die Hydrierung in mindestens zwei Reaktoren durchgeführt, besonders bevorzugt in mindestens einem Kreislaufreaktor und in mindestens einem Reaktor, der im geraden Durchgang betrieben wird. Vorzugsweise ist der Kreislaufreaktor dabei der erste Reaktor und der im geraden Durchgang betriebene Reaktor der zweite Reaktor.

Die Hydrierung ist eine exotherme Reaktion, bei der Wärme freigesetzt wird. Diese Wärmeenergie würde durch ein einfaches Kühlen vernichtet werden. Es ist erfindungsgemäße daher bevorzugt, wenn die Reaktionswärme zur Wärmeintegration innerhalb des Verfahrens verwendet wird. In einer bevorzugten Ausführungsform der vorliegenden Erfindung wird die Reaktionswärme der Hydrierung zur Vorwärmung des eingesetzten Eduktstroms genutzt, indem die Temperatur am Kreislaufkühler über 100°C, bevorzugt über 140°C und besonders bevorzugt über 160°C eingestellt wird, um zur Wärmeintegration nutzbares Warmwasser oder Heizdampf passender Druckstufe zu erzeugen.

Die Aufarbeitung der durch die Totalhydrierung erhaltenen Reaktionsmischung kann anschließend mittels thermischer Trennung, insbesondere mittels Destillation erfolgen. Derartige Verfahren sind dem Fachmann geläufig.

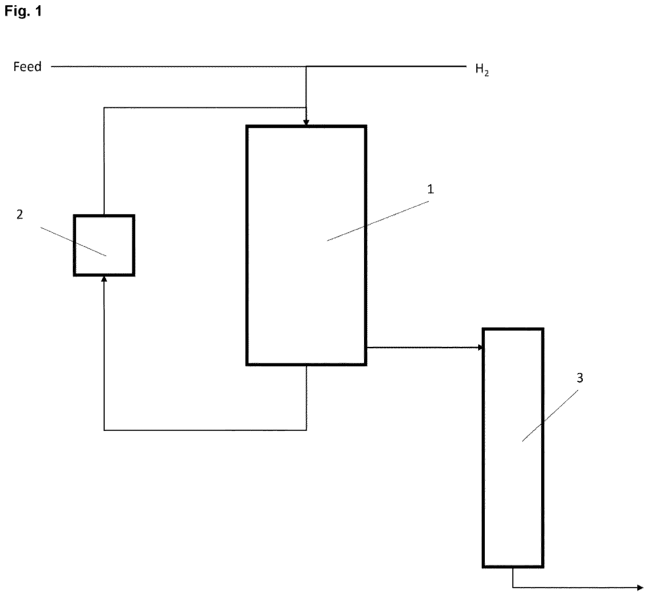

Eine bevorzugte Ausführungsform der vorliegenden Erfindung ist in Fig. 1 gezeigt. Diese Abbildung dient der Illustration und stellt keine Beschränkung des Erfindungsgegenstands dar. In Fig. 1 werden ein olefinhaltiger Kohlenwasserstoffstroms (Feed) und Wasserstoff (H₂) zu einem Reaktor (1) geführt, in dem sich der erfindungsgemäße Katalysator befindet. In dem im Kreislauf betriebenen Reaktor (1) findet die Hydrierung statt. Ein Teil des Reaktoraustrags wird über den Wärmetauscher (2) zurück zum Eingang des Reaktors (1) geführt. Der Wärmetauscher ist dann als Kreislaufkühler in dem Sinne ausgestaltet, dass der rezyklierte Strom abgekühlt wird, um damit zur Wärmeintegration nutzbares Warmwasser oder Heizdampf passender Druckstufe zu erzeugen. Ein anderer Teil des Reaktoraustrags wird zum Reaktor (2) geführt, wo ebenfalls ein erfindungsgemäßer Katalysator vorliegt. In dem im geraden Durchgang betriebenen Reaktor (2) werden restliche Olefine hydriert und es wird ein Produktstrom (4) erhalten.

### Beispiele

In einem zu Reaktor (1) äquivalentem Reaktor (Innendurchmesser 23 mm, Höhe der aktiven Katalysatorschüttung 41 cm) mit Kreislauf und Wärmeaustauscher im Kreislauf wurden Experimente zur Hydrierung durchgeführt. In den Experimenten wurde zunächst ein Katalysator mit Platin als aktives Metall eingesetzt. Anschließend wurde der Katalysator mit Platin gegen einen Katalysator mit Palladium ausgetauscht. Zum Vergleich wurden analoge Hydrierexperimente durchgeführt.

In einem Festbettreaktor (Innendurchmesser 23 mm, Höhe der aktiven Katalysatorschüttung 41 cm) mit zusätzlichem Kreislauf wurden Hydrierexperimente von analogen C12-Olefinen (Moleküle mit 12 Kohlenstoffatomen und jeweils einer Doppelbindung) durchgeführt. Dabei wurde in einem Experiment ein Katalysator 1 mit Platin (Pt) als aktivem Metall (0,5 Gewichtsprozent Pt auf Al₂O₃ - Extrudate mit Nenngröße 2 mm) und im vergleichenden Experiment ein Katalysator 2 mit Palladium (Pd) als aktivem Metall (0,5 Gewichtsprozent Pd auf Al₂O₃ - analoger Träger zum Katalysator 1 - Evonik Noblyst^{®} H14271) verwendet.

Die in Experimenten realisieren Versuchsparameter sind für Katalysator 1 (Pt) Tabelle 1 und für Katalysator 2 (Pd) Tabelle 2 zu entnehmen. Die Konzentration an Doppelbindungen ist über die angegebenen Bromzahlen (BrZ - Gramm Brom, die in einer Titration pro 100 g Probe verbraucht werden. Pro Mol Doppelbindung wird ein Mol Brom verbraucht.) zugänglich. Die BrZ gibt den Grad der Hydrierung an. Je geringer die Zahl umso weniger Doppelbindungen sind noch vorhanden. Zusätzlich ist in den Tabellen die sich ergebende Reaktionskonstante = ermittelte Geschwindigkeitskonstante der Hydrierreaktion bei der Temperatur am Anfang des aktiven Katalysators angegeben. Je höher dieser Wert ist, umso schneller läuft die Reaktion ab und desto höher die Aktivität des eingesetzten Katalysators.

**Tabelle 1: Versuchsparameter bei der Hydrierung mit Pt-Katalysator**

| Katalysator 1 | | |
|---|---|---|
| | Versuch 1 | Versuch 2 |
| Zulauf pro Menge Katalysator (kg / h*kg) | 5,4 | 5,5 |
| Druck H₂ (bar) | 13 | 12 |
| H₂ pro Olefin (mol/h / mol/h) | 1,1 | 1,1 |
| Temperatur am Anfang des aktiven Katalysators | 139 | 117 |
| Bromzahl im Reaktionsprodukt am Reaktorausgang | 0,15 | 0,91 |
| Reaktionskonstante | 12 | 8 |

**Tabelle 2: Versuchsparameter bei der Hydrierung mit Pd-Katalysator**

| Katalysator 2 | | |
|---|---|---|
| | Versuch 3 | Versuch 4 |
| Zulauf pro Menge Katalysator (kg / h*kg) | 6,3 | 6,0 |
| Druck H₂ (bar) | 14 | 13 |
| H₂ pro Olefin (mol/h / mol/h) | 1,2 | 1,1 |
| Temperatur am Anfang des aktiven Katalysators | 141 | 154 |
| Bromzahl BrZ im Reaktionsprodukt am Reaktorausgang | 9,25 | 0,98 |
| Reaktionskonstante | 3 | 6 |

Aus den in beiden Tabellen angegebenen Werten kann abgeleitet werden, dass sowohl die Aktivität des Katalysators mit Pt als auch die Aktivität des Katalysators mit Pd mit steigender Temperatur zunimmt. Die ermittelten Reaktionskostanten steigen jeweils für jeden Katalysatortyp mit der Temperatur. Zusätzlich wurde erstaunlicherweise gefunden, dass die Aktivität des Katalysators mit Platin schon bei einer niedrigeren Temperatur höher ist als die des äquivalenten Katalysators mit Palladium. Generell ergibt sich bei gleicher Temperatur für den Katalysator mit Platin eine höhere Aktivität als für den Katalysator mit Palladium.

## Patentansprüche

1. Heterogener Katalysator für die Totalhydrierung eines olefinhaltigen Kohlenwasserstoffstroms,
wobei der Katalysator ein Trägermaterial, welches aus der Gruppe bestehend aus Aluminiumoxid, Siliziumdioxid wie Kieselsäure oder Kieselgel, Kieselgur, Alumosilikate einschließlich Zeolithe, Titandioxid, Aktivkohle und Mischungen daraus, ausgewählt wird, und
ein einziges katalytisch aktives Metall in einer Menge von 0,05 bis 1 Gew.-%, bezogen auf das Gesamtgewicht des Katalysators, umfasst
wobei das katalytisch aktive Metall Platin ist.

2. Heterogener Katalysator nach Anspruch 1, wobei der Katalysator Platin in einer Menge von 0,1 bis 0,3 Gew.-%, bezogen auf das Gesamtgewicht des Katalysators, umfasst.

3. Heterogener Katalysator nach Anspruch 1 oder 2, wobei das Trägermaterial aus der Gruppe bestehend aus Siliciumdioxid, Aluminiumoxid, Titandioxid und Mischungen daraus ausgewählt wird.

4. Heterogener Katalysator nach einem der Ansprüche 1 bis 3, wobei das Platin in den Katalysatorpartikeln in einer äußeren Randzone vorliegt, wobei die Dicke der Randzone kleiner als 500 µm, vorzugsweise kleiner als 250 µm ist.

5. Verfahren zur Totalhydrierung eines olefinhaltigen Kohlenwasserstoffstroms bei dem mindestens 99 % aller Olefine im olefinhaltigen Kohlenwasserstoffstrom hydriert werden, wobei das Verfahren in einem Reaktor unter Zusatz von Wasserstoff durchgeführt wird, **dadurch gekennzeichnet, dass** in dem Verfahren der heterogene Katalysator nach einem der Ansprüche 1 bis 4 eingesetzt wird.

6. Verfahren nach Anspruch 5, wobei die Hydrierung in einem Rieselbettreaktor, in einem Kreislaufreaktor mit einem nachgeschaltetem Finisherreaktor oder in einem Pulse-Flow-Reaktor durchgeführt wird.

7. Verfahren nach Anspruch 5 oder 6, wobei die Reaktionswärme der Hydrierung zur Vorwärmung der Edukte genutzt wird, indem die Temperatur am Kreislaufkühler über 100°C, bevorzugt über 140°C und besonders bevorzugt über 160°C eingestellt wird, zur Wärmeintegration nutzbares Warmwasser oder Heizdampf passender Druckstufe zu erzeugen.

8. Verfahren nach einem der Ansprüche 5 bis 7, wobei der Wasserstoff bei der erfindungsgemäßem Totalhydrierung vorzugsweise in einer Menge von 1,0 bis 1,5 mol Wasserstoff pro mol Olefin zudosiert wird.

9. Verfahren nach einem der Ansprüche 5 bis 8, wobei der Wasserstoff bei der Totalhydrierung vorzugsweise in einer Menge von 1,0 bis 1,3 mol Wasserstoff pro mol Olefin zudosiert wird.

10. Verfahren nach einem der Ansprüche 5 bis 8, wobei der Wasserstoff bei der Totalhydrierung vorzugsweise in einer Menge von 1,0 bis 1,1 mol Wasserstoff pro mol Olefin zudosiert wird.

11. Verfahren nach einem der Ansprüche 5 bis 10, wobei der olefinhaltige Kohlenwasserstoffstrom ein olefinhaltiger Kohlenwasserstoffstrom ist, der lineare oder verzweigte Butene umfasst (C4-Ströme), der lineare oder verzweigte Dibutene umfasst (C8-Ströme), der lineare oder verzweigte Tributene umfasst (C12-Ströme) oder der lineare oder verzweigte Tetrabutene umfasst (C16-Ströme).

12. Verfahren nach einem der Ansprüche 5 bis 11, wobei die Totalhydrierung bei einer Temperatur zwischen 20 und 300 °C wird.

13. Verfahren nach einem der Ansprüche 5 bis 12, wobei bei der Totalhydrierung mehr als 99,5 % aller Olefine im olefinhaltigen Kohlenwasserstoffstrom hydriert werden.
